Europäisches Patentamt

**European Patent Office**

**Office européen des brevets**

(11) Publication number: **0 190 033 B1**

# EUROPEAN PATENT SPECIFICATION

(45) Date of publication of patent specification: **07.04.93**

(51) Int. Cl.5: **C07K 15/00**, C12P 21/00, C12N 5/00, C12N 15/00, G01N 33/577, G01N 33/574, //(C12P21/00,C12R1:91)

(21) Application number: **86300568.2**

(22) Date of filing: **28.01.86**

(54) **Monoclonal antibody against a ras oncogene P21 related dodecapeptide.**

(30) Priority: **29.01.85 US 696197**

(43) Date of publication of application:
**06.08.86 Bulletin 86/32**

(45) Publication of the grant of the patent:
**07.04.93 Bulletin 93/14**

(84) Designated Contracting States:
**AT BE CH DE FR GB IT LI LU NL SE**

(56) References cited:
**EP-A- 0 108 564**
**EP-A- 0 175 360**
**WO-A-84/01389**
**WO-A-85/00807**

**PROCEEDINGS OF THE NATIONAL ACADEMY OF SCIENCES OF USA, vol. 81, August 1984, pages 5227-5231; P. HORAN HAND et al.: "Monoclonal antibodies of predefined specificity detect activated ras gene expression in human mammary and colon carcinomas**

(73) Proprietor: **Oncogene Science, Inc.**
**106 Charles Lindbergh Boulevard**
**Uniondale, NY 11553-3649(US)**

(72) Inventor: **Carney, Walter Patrick**
**15 Anselm Terrace**
**Brighton Massachusetts 02135(US)**

(74) Representative: **Hildyard, Edward Martin et al**
**Frank B. Dehn & Co. European Patent Attorneys Imperial House 15-19 Kingsway**
**London WC2B 6UZ (GB)**

Rank Xerox (UK) Business Services
(3.10/3.5x/3.0.1)

**Description**

FIELD OF INVENTION

This invention concerns a murine monoclonal antibody that is immunoreactive with an antigen common to a variety of malignant cells, particularly lung, breast and colon carcinoma and a variety of hemopoietic tumors. Also of concern is the hybridoma cell line that secretes the antibody and diagnostic processes that employ the antibody or antibody fragment of the invention.

BACKGROUND OF THE INVENTION

The immune response to entry of a foreign substance into the body consists of secretion by plasma cells of "antibodies" which are immunoglobulin (Ig) molecules with combining sites that recognize particular determinants on the surface of the foreign substance, or antigen, and bind specifically to them. Immunoglobulin is the generic name for various isotypes of antibodies that include IgG, IgM, IgA, IgE, and the like. The various species of Ig have similiarities and differences. For example, all immunoglobulin molecules have a constant portion, i.e. highly conserved (constant) amino acid sequence, within a particular Ig subclass (e.g., $IgG_1$). This constant region is responsible for various biological effector functions (e.g., complement activation). The portion of the immunoglobulin molecule responsible for immunological specificity (i.e., specific antigen binding) is called the variable region. It is made up of the variable regions of the Ig heavy and light chains. These variable regions differ in amino acid sequence according to the antigenic determinant which the Ig recognizes. Usually, the antibody (Ab) response to an antigen (Ag) is heterogeneous. Upon injection of a body with an immunogen, the body manufactures large numbers of antibodies directed against various components of the antigens and even against various determinant sites on the antigen. It is difficult to separate antibodies from conventional antisera containing mixtures of antibodies. It has, therefore, long been a goal to design a continuous source of defined antibodies that recognize and combine with specific antigen determinants.

Hybridoma technology concerns the fusion of myeloma cells (parent cells) with lymphocytes from animals which have been immunized with a particular antigen. The resulting hybridoma cell manufactures monoclonal antibodies that are specific against a single antigenetic determinant. Monoclonal antibodies are beginning to replace conventional antisera in standard diagnostic kits for such procedures as the radioimmunoassay. Significant work is also being done to adapt hybridoma technology for therapeutic purposes.

A typical procedure for making hybridomas is as follows:
(a) immunize mice with a certain immunogen; (b) remove the spleens from the immunized mice and make a spleen suspension in an appropriate medium; (c) fuse the suspended spleen cells with mouse myeloma cells; (d) dilute and culture in separate containers the mixture of unfused spleen cells, unfused myeloma cells and fused cells in a selective medium which will not support growth of the unfused myeloma cells; (e) evaluate the supernatant in each container containing hybridoma for the presence of antibody to the immunogen; and (f) select and clone hybridomas producing the desired antibodies. Once the desired hybridoma has been selected and cloned, the resultant antibody is produced by in vitro culturing of the desired hybridoma in a suitable medium. In an alternative method, the desired hybridoma can be injected directly into mice to yield concentrated amounts of antibody.

Hybridomas produced by fusion of murine spleen cells and murine myeloma cells have been described in the literature by Kohler et al., in Eur. J. Immunol. 6, 511-519 (1976); by Milstein et al. in Nature 266, 550 (1977); and by Walsh, Nature, 266, 495 (1977).

The technique is also set out in some detail by Herzenberg and Milstein, in "Handbook on Experimental Immunology", ed. Weir (Blackwell Scientific, London), 1979, pages 25.1 to 25.7. Additional references for hybridoma methodology are provided in "Monoclonal Antibodies" Plenum Press, 1980, R. Kennett, J. McKearn, and K. Bectol eds.

Patents relating to monoclonal antibodies against human tumors produced by hybridoma technology include U.S. Patents 4,182,124 and 4,196,265. Representative of the art concerning monoclonal antibodies that have specificity for antigens on carcinoma cells are U.S. patent 4,349,528 and U.S. patent 4,350,683.

Relative to the parent myeloma cell line employed herein for the fusion event, see Kearney et al, Immunol., 123, 1548-1550 (1978).

DNA mediated transfection experiments using NIH3T3 cells as recipients have led to the identification of human transforming genes from a wide variety of tumor types including both established cell lines and primary tumor tissues from cancer patients. To date, approximately 20% of all tumor cells tested have been found to contain transforming genes, termed activated ras genes. The ras genes present in mammalian cells have been demonstrated to be homologous to murine sarcoma viral oncogenes. Thus, ras retroviral oncogenes have been found in tumors as diverse as carcinomas, sarcomas, neuroblastomas and hematopoietic malignancies.

Ras genes are found in all nucleated mammalian cells and encode a 21,000 molecular weight (P21) intracellular membrane associated protein. Ras gene products (P21) have been found in a wide variety of neoplastic cells and have been shown to differ structurally from ras gene P21 products found in normal cells. These structural alterations have been demonstrated to be amino acid substitutions at positions 12 and 61 of the 189 amino acid long ras gene product. The P21 protein found in normal cells has the following primary amino acid structure for amino acid residues 5 through 16: Lysine-Leucine-Valine-Valine-Valine-Glycine-Alanine-Glycine-Glycine-Valine-Glycine-Lysine. In contrast, neoplastic cells such as the T24 bladder carcinoma have been shown to have an amino acid substitution such as valine at position 12 with amino acid residues 5,6,7,8,9,10,11,13,14,15 and 16 being identical to those found in P21 molecules in untransformed cells. In addition, other neoplastic cells have been shown to contain amino acid substitutions other than valine at position 12.

The subject of this invention is the induction, production and characterization of a monoclonal antibody that reacts with mutant amino acids at position 12 and that does not react with the glycine amino acid normally present at position 12. An antibody such as DWP, described in this invention is a valuable diagnostic tool for the detection of primary and metastatic neoplastic cells.

In one aspect our invention provides a monoclonal antibody which binds specifically to an epitope of an activated ras protein containing valine at position 12 and does not bind to an epitope of normal nononcogenic ras protein containing glycine at position 12, this epitope of the activated ras protein being characterized in that it is capable of being bound by a monoclonal antibody produced by hybridoma cell line having ATCC Accession Number 8698.

Our invention also extends to immunoreactive fragments of the monoclonal antibodies defined above, since for many purposes these fragments will serve as well as the intact antibody.

Monoclonal antibodies according to our invention may be made, as described more fully hereinafter, by culturing suitable hybridomas.

The hybridoma cell line which was found to secrete the preferred monoclonal antibody of the subject invention was deposited in the ATCC with the accession number HB-8698, and is hereafter referred to as DWP.

A typical procedure for producing the monoclonal antibody of our invention will now be described.

A Balb/c x C57BL/6 mouse was immunized on several occasions with a synthetic dodecapeptide identical in primary amino acid structure to that of amino acid residues number 5 through 16 of the ras oncogene protein product P21 found in the T24 bladder carcinoma cells. The structure of the peptide (linked to bovine thyroglobulin for immunization) was the following: Lysine-Leucine-Valine-Valine-Valine-Glycine-Alanine-Valine-Glycine-Valine-Glycine-Lysine. Spleen cells from mouse 1637 were fused with Sp2/0 mouse myeloma cells and two weeks later culture supernatants were screened by enzyme-linked immunosorbent assay. The hybridoma designated DWP was selected because of its reactivity on the immunizing peptide described above and because of its lack of reactivity with a peptide identical in primary amino acid structure to residue 5 to residue 16 of the ras protooncogene containing glycine at position 12 (i.e., Lysine-Leucine-Valine-Valine-Valine-Glycine-Alanine-Glycine-Glycine-Valine-Glycine-Lysine). The DWP monoclonal antibody shows specificity for dodecapeptides containing valine or cysteine at position 12 and is unreactive with dodecapeptides containing glycine, aspartic acid, glutamic acid, arginine, serine or alanine at position 12 of the ras P21 molecule. Normal and neoplastic cells were surveyed by the avidin-biotin immunoperoxidase technique to determine the clinical utility of the antibody. The immunoreactive antigen recognized by this antibody was found to be present in lung, breast and colon carcinomas as well as hematopoietic tumors but unreactive with normal colon, lung, spleen, heart or liver tissue.

## DETAILED DESCRIPTION OF THE INVENTION

### Immunizations

A Balb/c C57B1/6 mouse designated 1637 was immunized intraperitoneally (I.p) with 100 $\mu$g of bovine thyroglobulin (Btg) conjugated to a dodecapeptide having the following primary amino acid structure: Lysine-Leucine-Valine-Valine-Valine-Glycine-Alanine-Valine-Glycine-Valine-Glycine-Lysine (hereinafter "peptide 1"). The first inoculation of Btg-peptide was given in complete Freunds Adjuvant on day 1 and subsequent inoculations of 100 $\mu$g of immunogen were given on days 14, 28, 42. On day 59, 3 days before fusion, mouse 1637 was given an I.p. boost of 200 $\mu$g of immunogen.

### Hybridoma Methodology

Three days after an intraperitoneal boost (day 59), the spleen of mouse 1637 was removed and fused with the non-secretor myeloma cell Sp2/0. More particularly, after sacrifice of mouse 1637, spleen cell suspensions were prepared in serumless DMEM-high glucose medium, and mixed with myeloma cells at a ratio of 4:1. This cell mixture was centrifuged at 1200xg for 10 minutes at room temperature. After removal of the supernatant, the cells were resuspended by gently tapping the tube. The fusion procedure was initiated by adding 1.0 ml of 45% w/v polyethylene glycol 3350 (Baker) at 37°C over a 30 second period.

The cells were occasionally mixed with a pipette tip for 90 seconds and 5 ml of serumless DMEM-high glucose medium was added over a 3 minute period. This was followed by the addition of 14 ml of DMEM-high glucose supplemented with 10% fetal calf serum, L-glutamine, hypoxanthine, aminopterin and thymidine (referred to as HAT medium). The HAT medium was added over a 1 minute period.

Total volume was then adjusted to 50 ml with HAT medium and the cells were centrifuged at 800xg for 7 minutes at room temperature. Supernatants were aspirated and the cell pellet disrupted with 10 ml of HAT medium. $5 \times 10^6$ peritoneal cells (Balb/cx C57B1/6) were added and the final volume adjusted to 240 ml. Cells were then pipetted in to 96 well microtiter plates at a final concentration of $2 \times 10^5$ spleen cells per well. Approximately 14 days later tissue culture supernatants from wells containing hybridoma colonies were tested by enzyme-linked immunosorbent assay (ELISA) for the desired reactivity with peptides conjugated to carrier proteins.

### Screening Procedures and Elisa Protocol

For screening purposes peptide 1 was conjugated to carrier protein Keyhole Limpet Hemocyanin (KLH). Prior to screening hybridoma supernatants, 500 ng of the KLH carrier protein bound to peptide 1 was dispensed to 96-well microtiter plates (Immulon II) for overnight incubation at 37°C. After incubation, plates were washed and unbound sites were blocked with bovine serum albumin (BSA).

At the time of screening 50 µl of supernatant fluid from hybridoma DWP was added to wells containing carrier protein bound to peptide 1 and incubated overnight at 4°C. Hybridoma supernatant was removed on the next day, and wells were washed with a BSA solution. Each well subsequently received 50 µl of goat anti-mouse IgG antibody conjugated to horseradish peroxidase (GAMHRP) diluted in BSA phosphate buffered saline (PBS). Wells were incubated for 60 minutes at 37°C. GAMHRP was removed after incubation and wells were washed three times with PBS-BSA mixtures. The presence of bound GAMHRP was determined by adding 50 µl of the substrate o-phenylene diamine (OPD) in phosphate buffer containing 0.15% hydrogen peroxide. HRP in combination with its substrate (OPD) results in a yellow colored product. Development of the yellow product was allowed to occur at room temperature for 15 minutes. The enzymatic reaction was terminated by the addition of 50 µl of 4.5M $H_2SO_4$. Measurement of the resultant reaction product was accomplished by determining optical density (OD) at 488 nm. Presence of yellow color in the wells indicated that the DWP mouse antibody was present in the hybridoma supernatant. The DWP antibody was found to be an IgG2b kappa molecule by using rabbit anti-mouse immunoglublin reagents.

### Titration of the DWP Antibody on KLH-Peptide Conjugates

After the DWP hybridoma had been cloned by limiting dilution, it was grown in serumless media for 5 days and the DWP antibody concentrated by ammonium sulfate ($NH_4 SO_4$) precipitation.

The concentrated DWP antibody was then evaluated by ELISA for reactivity to KLH conjugated peptide 1, as well as peptides with glycine at position 12 (peptide 2) and cysteine at position 12 (peptide 3). The DWP antibody showed little or no reactivity (as indicated by measuring optical density at 488 nm) on peptide 2 whereas the DWP antibody showed reactivity at several dilutions of peptide 1 and peptide 3.

### Competition Assays

To evaluate DWP specificity, a series of assays were performed to determine whether unconjugated peptides containing various amino acid substitutions at position 12 could inhibit DWP binding to peptides 1 and 3. A 1:400 dilution of a 300 µg/ml solution of DWP was mixed with equal volume of PBS containing various concentrations of competitor (unconjugated peptides containing various amino acid substitutions at position 12). The competitor concentration ranged from 15.6 to 500 ng/well.

After a 1-2 hour incubation at 37°C the DWP antibody and competitor mixture was pipetted into microtiter wells containing 500 ng of the peptide 1 (valine at position 12) conjugated to KLH to assess the presence of unbound DWP antibody. Unbound DWP antibody would then bind peptide 1. Peptide 1 and peptide 3 both bound the DWP antibody. In contrast, several peptides containing glycine, aspartic acid, glutamic acid, alanine, serine or arginine at position 12 did not bind the DWP antibody. These experiments demonstrate that the DWP reactivity is specific for the immunogen peptide containing valine at position 12 and surprisingly is reactive with a peptide not used in the immunization containing cysteine at position 12. The DWP antibody was unreactive with series of peptides containing various amino acid substitutions as well as the carrier protein, KLH.

Immunohistochemistry By Immunoperoxidase Staining

Six micron tissue sections fixed with formalin and embedded in paraffin were used to evaluate the clinical usefulness of the DWP antibody.

Fixed tissues were deparaffinized in xylene for 30 minutes at 65°C followed by a 30 minute incubation in xylene at room temperature. Tissues were washed two times in 100% ethanol followed by two washes with 95% alcohol. Tissue sections were thoroughly rinsed in water followed by washing in PBS. Tissues received normal horse serum for a 30 minute incubation to block nonspecific sticking. The monoclonal antibody secreted by hybridoma DWP was applied (2 $\mu$g/slide) and incubated overnight at 4°C. After overnight incubation the tissue sections were washed 3 times in PBS and then incubated for 30 minutes with biotinylated horse anti-mouse reagents. Tissue sections were washed with PBS and incubated for 30 minutes with avidin-biotinylated horseradish peroxidase complex. Tissue sections were rinsed in PBS 3 times and reacted with diaminobenzidine and hydrogen peroxide for 5 minutes. The tissue sections were rinsed in PBS, counterstained with hematoxylin for 30 seconds, washed for 30 seconds in alcohol, washed for 30 seconds in ammonium sulfate reagent and dehydrated. Dehydration was carried out by dipping sections in 80% ethanol for 30 seconds, in 95% ethanol for 30 seconds, in 100% ethanol for 30 seconds with two changes in xylene for 10 minutes. Slides were then mounted and examined by microscope to determine the presence of a brownish precipitate which indicates the presence of DWP antibody. The tissue staining as determined by immunoperoxidase reactivity on a variety of normal and neoplastic tissues is summarized in Table 1 and 2, however the most notable observations are the following:

(1) positive immunoperoxidase reactivity on 4 out of 9 infiltrating ductal carcinomas of the breast and no reactivity on 1 out of 2 cases of fibrocystic disease.

(2) positive immunoperoxidase reactivity on 5 out of 6 large cell undifferentiated carcinomas of the lung.

(3) positive immunoperoxidase reactivity on 2 out 2 oat cell or small cell carcinomas of the lung.

(4) positive reactivity on primary lesion of oat cell carcinoma as well as positive reactivity of that oat cell carcinoma after its metastasis to the lymph node.

(5) positive immunoperoxidase reactivity on 7 of 8 colon adenocarcinomas and 4 of 6 villous adenomas of the colon.

(6) positive immunoperoxidase reactivity on 2 out of 2 endometrioid carcinomas.

(7) positive immunoperoxidase reactivity on a common bile duct carcinoma metastatic to the ovary.

(8) negative immunoperoxidase reactivity on apparently normal tissues adjacent to the above (1-7) described carcinomas and no reactivity on normal tissues listed on Table 2.

(9) all carcinoma tissues were negative in control studies whereby the MOPC 141 IgG2b class matched antibody control was substituted for the DWP antibody at the same antibody concentration.

(10) analysis of cell line PSV-13 (overexpression of ras P21 protein containing the normal amino acid glycine at position 12) by immunoperoxidase staining indicated no staining by the DWP antibody. In contrast, a cell line designated PSV-LM (EJ) containing high overexpression of the mutant ras P21 protein containing valine at position 12 was strongly immunoreactive with the DWP antibody.

## TABLE 1

### Reactivity Of The DWP Antibody On Neoplastic Tissues By Immunoperoxidase Staining

| Tissue | # Positive/Total # |
|---|---|
| **Breast** | |
| Infiltrating Ductal Carcinoma | 4/9 |
| Intraductal Carcinoma | 0/2 |
| Fibrocystic Disease | 1/2 |
| **Lung** | |
| Large Cell Undifferentiated | 5/6 |
| Squamous Cell Carcinoma | 0/2 |
| Adenocarcinoma | 0/1 |
| Oat Cell Carcinoma | 2/2 |
| Oat Cell Metastatic to Lymph Node | 1/1 |
| **Colon** | |
| Colon Adenocarcinoma | 7/8 |
| Villous Adenoma | 4/6 |
| **Ovarian** | 0/2 |
| Endometrioid Carcinoma | 2/2 |
| Metastatic Carcinoma from Common Bile Duct | 1/1 |

Apparently normal tissue adjacent to tumor cells were unreactive with DWP.
The MOPC 141 class-matched IgG2b control was unreactive on tissue sections.

### TABLE 2

| Formalin-Fixed Paraffin Embedded Normal Tissues and Immunoperoxidase Staining with Antibody DWP ||
|---|---|
| Tissue Source | # Positive/Total # |
| Colon | 0/8* |
| Stomach | 2/2* |
| Spleen | 0/10 |
| Lung | 0/10* |
| Liver | 0/10 |
| Heart | 0/10 |

*Some immunoperoxidase reactivity on smooth muscle.

The monoclonal antibody DWP is useful in the diagnosis of primary and metastatic lesions by conventional diagnostic methods. More specifically, the DWP antibody can be used to detect primary carcinomas, including breast, lung and colon cells as well as metastasis to lymph nodes and other organs of the body.

Diagnosis may also be carried out by conventional in vitro diagnostic procedures such as the assay of human blood samples or other bodily fluids. In addition, diagnosis may also be carried out by the evaluation of human tissue sections using this antibody and immunohistochemical techniques of immunoperoxidase staining; and the detection of micro-lesions containing only a few tumor cells that would not ordinarily be detected by conventional staining techniques is now possible using the monoclonal antibody of this invention.

**Claims**

1. A monoclonal antibody which binds specifically to an epitope of an activated ras protein containing valine at position 12 and does not bind to an epitope of normal nononcogenic ras protein containing glycine at position 12, this epitope of the activated ras protein being characterized in that it is capable of being bound by a monoclonal antibody produced by hybridoma cell line having ATCC Accession Number 8698.

2. A hybridoma that secretes a monoclonal antibody of claim 1.

3. The hybridoma designated ATCC HB-8698.

4. Monoclonal antibody secreted by the hybridoma of claim 3.

5. An immunoreactive fragment of the antibody of claim 1 or claim 4.

6. A process for the preparation of a monoclonal antibody reactive with a peptide containing the amino acid sequence Lysine-Leucine-Valine-Valine-Valine-Glycine-Alanine-Valine-Glycine-Valine-Glycine-Lysine which comprises culturing the hybridoma defined in claim 2 and recovering the monoclonal antibody so produced.

7. The process of claim 6 wherein the hybridoma defined in claim 3 is cultured.

8. A process for the preparation of a hybridoma as defined in claim 2 which comprises immunizing an animal with a peptide containing the amino acid sequence defined in claim 6, fusing spleen cells from said immunized animal with non-immunoglobulin secreting myeloma cells, and selecting from the resulting hybridomas a cell line which secretes a monoclonal antibody having the desired binding specificity.

**Patentansprüche**

1. Ein monoklonaler Antikörper, welcher spezifisch an ein Epitop eines aktivierten RAS-Proteins, enthaltend Valin in Stellung 12, bindet und nicht an ein Epitop eines normalen nicht-oncogenen RAS-Proteins, enthaltend Glycin in Stellung 12, bindet, wobei dieses Epitop des aktivierten RAS-Proteins dadurch gekennzeichnet ist, daß es in der Lage ist, durch einen monoklonalen Antikörper gebunden zu sein, der durch eine Hybridoma-Zellinie mit der ATCC-Eintragungsnummer 8698 hergestellt ist.

2. Hybridoma, welches einen monoklonalen Antikörper gemäß Anspruch 1 absondert.

3. Das Hybridoma, bezeichnet mit ATCC HB-8698.

4. Monoklonaler Antikörper, abgesondert durch das Hybridoma gemäß Anspruch 3.

5. Ein immunoreaktives Fragment des Antikörpers des Anspruchs 1 oder Anspruchs 4.

6. Verfahren zur Herstellung eines monoklonalen Antikörpers, der mit einem Peptid reaktiv ist, das die Aminosäure-Sequenz Lysin-Leucin-Valin-Valin-Valin-Glycin-Alanin-Valin-Glycin-Valin-Glycin-Lysin enthält, welches das Züchten des in Anspruch 2 definierten Hybridomas und Gewinnung des so hergestellten monoklonalen Antikörpers umfaßt.

7. Verfahren gemäß Anspruch 6, worin das in Anspruch 3 definierte Hybridoma gezüchtet wird.

**8.** Verfahren zur Herstellung eines Hybridomas, wie in Anspruch 2 definiert, welches umfaßt das Immunisieren eines Tieres mit einem Peptid, enthaltend die in Anspruch 6 definierte Aminosäure-Sequenz, das Verschmelzen von Milzzellen aus diesem immunisierten Tier mit Nicht-Immunoglobulin abscheidenden Myeloma-Zellen und Selektieren einer Zellinie aus den resultierenden Hybridomas, welche einen monoklonalen Antikörper mit der gewünschten Bindungsspezifizität abscheidet.

**Revendications**

**1.** Anticorps monoclonal, qui se fixe spécifiquement à un épitope d'une protéine ras activée contenant la valine en position 12 et ne se fixe pas à un épitope d'une protéine ras normale non oncogène contenant la glycine en position 12, cet épitope de la protéine ras activée étant caractérisé en ce qu'il est capable d'être fixé par un anticorps monoclonal produit par une lignée de cellules d'hybridome ayant le numéro d'accès ATCC 8698.

**2.** Hybridome qui sécrète un anticorps monoclonal suivant la revendication 1.

**3.** L'hybridome dénommé ATCC HB-8698.

**4.** Anticorps monoclonal sécrété par l'hybridome suivant la revendication 3.

**5.** Fragment immunoréactif de l'anticorps suivant la revendication 1 ou la revendication 4.

**6.** Procédé pour la préparation d'un anticorps monoclonal réagissant avec un peptide contenant la séquence d'acides aminés Lysine-Leucine-Valine-Valine-Valine-Glycine-Alanine-Valine-Glycine-Valine-Glycine-Lysine, qui comprend la culture de l'hybridome suivant la revendication 2 et la récupération de l'anticorps monoclonal ainsi produit.

**7.** Procédé suivant la revendication 6, dans lequel l'hybridome suivant la revendication 3 est cultivé.

**8.** Procédé pour la préparation d'un hybridome suivant la revendication 2, qui comprend l'immunisation d'un animal avec un peptide contenant la séquence d'acides aminés définie dans la revendication 6, la fusion de cellules de rate provenant de cet animal immunisé avec des cellules de myélome ne sécrétant pas d'immunoglobulines et la sélection à partir des hybridomes résultants d'une lignée cellulaire qui sécrète un anticorps monoclonal ayant la spécificité de liaison désirée.